(19) Europäisches Patentamt
European Patent Office
Office européen des brevets

(11) Publication number: **0 088 383**
**B1**

(12) **EUROPEAN PATENT SPECIFICATION**

(45) Date of publication of patent specification: **15.01.86**

(21) Application number: **83102099.5**

(22) Date of filing: **03.03.83**

(51) Int. Cl.$^4$: **C 07 C 43/17,** C 07 C 43/215, C 07 C 41/16, C 07 C 41/30

(54) **Process for preparing the compound 1-methoxy-6-chloro-hexyne-2.**

(30) Priority: **04.03.82 IT 1995682**

(43) Date of publication of application:
**14.09.83 Bulletin 83/37**

(45) Publication of the grant of the patent:
**15.01.86 Bulletin 86/03**

(84) Designated Contracting States:
**BE CH DE FR GB LI NL**

(56) References cited:
**EP-A-0 037 092**

**CHEMICAL ABSTRACTS, vol. 81, no. 13, 30th September 1974, page 421, no. 77432x, Columbus, Ohio, USA P.E. PETERSON et al.: "4-Chloro-4-hexenyl trifluoroacetate"**

(73) Proprietor: **Montedison S.p.A.**
**31, Foro Buonaparte**
**I-20121 Milan (IT)**

(72) Inventor: **Massardo, Pietro, Dr.**
**5, via Fra'Bartolomeo**
**I-Milano (IT)**
Inventor: **Bettarini, Franco, Dr.**
**4/B, Via Cadore**
**I-Novara (IT)**
Inventor: **Bianchini, Ennio**
**8, Via Paletta**
**I-Novara (IT)**
Inventor: **Piccardi, Paolo**
**8, Via E. De Marchi**
**I-Milano (IT)**

(74) Representative: **Barz, Peter, Dr. et al**
**Patentanwälte Dr. V. Schmied-Kowarzik Dipl.-Ing. G. Dannenberg Dr. P. Weinhold Dr. D. Gudel Dipl.-Ing. S. Schubert Dr. P. Barz**
**Siegfriedstrasse 8**
**D-8000 München 40 (DE)**

**EP 0 088 383 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a process for the preparation of 1 - methoxy - 6 - chloro - hexyne - 2 (compound I) and, more particularly, it relates to a process for synthetising said compound consisting in condensing methyl - propargyl - ether with 1 - bromo - 3 - chloro - propane.

In Org. Syn. *1973*, 53, 1868 (C.A. 81, 77432x) a process is described wherein propyne is sequentially reacted with Na and $Br(CH_2)_3Cl$ in $NH_3$ at −73°C to give 6 - chloro - hexyne - 2. Besides the relatively low yield (38%), this process also has to be carried out at an inconvenient temperature. Moreover, the effect of the presence of a methoxy group in the propyne on the course of the reaction and the yield can not be predicted.

Compound I is a valuable intermediate for the production of compounds endowed with high acaricide activity, e.g. 1 - decyloxy - 4 - [(7 - oxa - 4 - octynyl) - oxy] - benzene as described in EP—A—37 092.

According to said reference, compound I may be prepared by reacting propargyl methyl ether with a strong base such as butyl lithium and reacting the lithium salt thus obtained with 3-chloro (or 3 - bromo) - 1 - propanol in which the hydroxy group has previously been protected, for instance in the form of an acetal. After deprotection, the OH-group of the resulting alcohol is replaced by a chlorine atom by conventional techniques.

Said process exhibits various drawbacks which render it not very useful to be utilized on an industrial scale.

These drawbacks result from the necessity of carrying out expensive operations, such as protection and deprotection of the hydroxy group and replacement of the hydroxy group by a chlorine atom.

Moreover, these and other reactions require the use of expensive and/or dangerous reagents which are practically impossible to recover.

Furthermore, due to the fact that the process consists of a number of steps and that it is necessary to isolate some of the resulting intermediates, the process yields are not particularly high.

The process according to the present invention overcomes the above mentioned problems.

The corresponding reaction may be represented by the following scheme:

$$CH_3—O—CH_2—C{\equiv}C^-Na^+ + Br—CH_2—CH_2—CH_2—Cl \longrightarrow$$

$$CH_3—O—CH_2—C{\equiv}C—CH_2—CH_2—CH_2—Cl + NaBr \qquad (I)$$

Methyl-propargyl-ether and 1-bromo-3-chloro-propane are known, commercially available compounds.

The reaction is conducted in a solvent mixture composed of tetrahydrofuran (THF) and of an aprotic polar solvent in amounts ranging from 5 to 50% by volume, based on THF, preferably from 10 to 30%.

The preferred aprotic polar solvents are dimethylsulphoxide (DMSO) and hexamethylene-phosphorus triamide (HMPA).

It should be noticed that without the presence of the aprotic polar solvent, the reaction does not occur at all or takes place with non-significant yields.

Since the above reaction is exothermic, it is preferably operated at temperatures of approximately 0°C (from −10° to +10°C), allowing the temperature to rise, at a later state, up to room temperature.

The sodium salt of methyl-propargyl-ether ($CH_3O—CH_2—C{\equiv}C^-Na^+$) is preferably prepared in situ by reacting, in anhydrous THF, methyl-propargyl-ether (in a concentration of from 0.5 to 1.5 molar) with sodium amide ($NaNH_2$), the forming of the salt being followed by detecting the evolved ammonia amount.

To the solution of the sodium salt in THF, the concentration of which is preferably 1 molar, is then added a predetermined amount of aprotic polar solvent and 1 - bromo - 3 - chloro - propane or preferably a solution of 1 - bromo - 3 - chloro - propane in the aprotic polar solvent.

In accordance with the stoichiometry of the reaction, 1 - bromo - 3 - chloro - propane and the sodium salt of methyl - propargyl - ether must be employed in substantially equimolar amounts. A slight excess of sodium salt can be optionally used.

By operating according to the foregoing, compound I is obtained in high yields (70—85%).

In a practical embodiment, the reaction is accomplished by operating as follows.

A predetermined amount of methyl-propargyl-ether is added to a suspension, in anhydrous THF, of a $NaNH_2$ amount ranging from 1 to 2 moles per mole of ether, at a temperature of about 10—30°C.

Ammonia evolution is followed until full conversion of ether into the corresponding sodium salt is reached.

This operation can be carried out by indirect titration by bubbling the ammonia in a $H_2SO_4$ solution of known titre and by re-titration of the latter, or by volumetrically measuring the evolved $NH_3$ which is then removed by acidified water.

To the solution of the sodium salt in THF, cooled down to −10° to 0°C is added dropwise a solution of a substantially equimolar amount, with respect to the sodium salt, of 1 - bromo - 3 - chloro - propane in a predetermined amount of DMSO and/or of HMPA.

The reaction exothermy is controlled and, upon completion of the addition, the temperature is allowed to rise up to about 10°C. The reaction mixture is then treated according to conventional techniques. For example, the mixture is poured into water and is extracted with an organic solvent.

Crude compound I is obtained by removal of the solvent from the organic extract, the product being employable for successive reactions either as such or after purification by distillation.

As compared with the process of EP—A—37 092 the process forming the object of the present invention offers several advantages, which can be summarized as follows:

— simple feasibility: the process of the invention is carried out in one step only and does not require the isolation of an intermediate; the process of EP—A—37 092 must be carried out in at least four steps and requires the isolation of several intermediates.
— economy: the organic reagents employed in the process according to this invention directly serve to the production of compound I; in contrast, in the process of EP—A—37 092 it is necessary to employ organic reagents for different purposes, such as dihydropyran or equivalents as a protective group, and p - toluene - sulphonyl - chloride to transform the hydroxy group of the alcohol into a good leaving group;
— better industrial suitability: the process of this invention requires, due to its simple feasibility and to the fact that it comprises only one step, less expensive plants and lower investment costs, and it can be easily converted to a continuous process; the process according to EP—A—37 092, since it comprises at least four steps and requires the isolation of intermediates, necessitates plants of larger dimensions and consequently higher investment costs;
— less pollution problems: the waste product of the process according to this invention is NaBr, which does not raise problems connected with the environmental pollution as they are encountered with the waste products of the process according to EP—A—37 092 (e.g. dihydropyran and sodium p - toluene - sulphonate), which, besides, are recoverable only at high costs;
— high yields: the process of EP—A—37 092 provides low yields as it comprises several steps, none of which being quantitative, and requires the isolation of intermediates, with consequent yield reduction. The process of the present invention provides yields of 70—85%.

The following examples are given to better illustrate the present invention.

Example 1

Preparation of 1-methoxy-6-chloro-hexyne-2 (compound I)

$$CH_3O—CH_2—C\equiv C—CH_2—CH_2—CH_2—Cl$$

12.5 g (0.10 moles) of methyl-propargyl-ether ($CH_3O—CH_2C\equiv CH$) were gradually added to a suspension of 8 g (0.205 moles) of sodium amide ($NaNH_2$) in 170 ml of anhydrous THF, maintained under stirring at room temperature.

The evolution of ammonia was monitored. It was moderately heated (30°C) until the evolution of the theoretical amount of ammonia was complete, whereafter it was cooled to 0°C and maintained under stirring at this temperature while a solution of 28.35 g (0.18 moles) of 1 - bromo - 3 - chloro - propane ($Br—CH_2—CH_2—CH_2—Cl$) in 28 ml of DMSO (16.5% by volume based on THF) was added dropwise.

The reaction was exothermic. On completion of the addition and of the exothermy, the temperature was allowed to rise up to 10°C, while stirring was continued for 30 minutes.

The reaction mixture was then poured into water and extracted with diethyl ether (3×100 ml).

The ethereal extracts were combined, washed with water and dried over anhydrous $Na_2SO_4$. The solvent was then removed by evaporation under reduced pressure, thus obtaining a crude product (26 g) substantially consisting of compound I.

The crude product was purified by distillation under reduced pressure, and the fraction boiling at 56°C at a pressure of 2.7 mbar (2 mm Hg) was collected, thus obtaining 19.9 g of compound I, which, subjected to gas-chromatography, revealed a purity degree of 90% (Yield based on the purified product: 76%).

$^1$H-NMR ($CDCl_3$, TMS)
δ(ppm): 1.95 (m, 2H, $—CH_2—CH_2Cl$)
2.4 (m, 2H, $\equiv C—CH_2—CH_2$)
3.3 (s, 3H, $CH_3$)
3.7 (t, 2H, $CH_2—Cl$)
4 (t, 2H, $O—CH_2$, J=0.2)

[s=singlet, t=triplet, m=multiplet or unresolved coupled signal, J=coupling constant]

Example 2

Preparation of compound I was repeated by operating in a similar manner as described in example 1, but varying some parameters. The results of these tests (Nos. 1—5) and of two comparative tests (Nos. 6 and 7) carried out as in example 1 but without using the aprotic polar solvent, are listed in the following table. From the tests reported in said table it is apparent that the aprotic polar solvent plays a critical role as

in its absence the reaction does not provide appreciable yields even if subjected to heating, the 1 - bromo - 3 - chloro - propane being recovered practically quantitatively.

TABLE
Preparation of Compound I

| Test No. | Propargyl-ether/ bromo-chloro-propane (molar ratio) | DMSO/THF (% by volume) | HMPA/THF (% by volume) | Reaction time (hours) | Final temperature (1) | Pure compound I yield (2) |
|---|---|---|---|---|---|---|
| 1 | 1/1 | 14% | — | 1 | 10°C | 75% |
| 2 | 1.5/1 | 15% | — | 1 | 10°C | 85% |
| 3 | 1/1 | 10% | — | 1 | 10°C | 70% |
| 4 | 1/1 | — | 10 | 1 | 10°C | 74% |
| 5 | 1/1 | 30% | — | 1 | 10°C | 80% |
| 6 (3) | 1/1 | — | — | 24 | 10°C | 0% |
| 7 (4) | 1/1 | — | — | 24 | 60°C | <2% |

Notes:
(1) Reaction started at about −10°C as it was exothermic.
(2) Yields refer to compound I, isolated and purified by distillation, and are based on the employed bromo-chloro-propane.
(3) Comparative test without aprotic polar solvent. After 1 hour and 4 hours at a temperature of 10°C, no formation of compound I was observed (gas-chromatographic check). The reaction mixture was left under stirring up to 24 hours whereby analogous results were obtained.
(4) Comparative test carried out at 60°C to ascertain that the unsatisfactory results of test No. 6 did not result from the low temperature. After 24 hours at 60°C, the gas-chromatographic check revealed compound I yields lower than 2%.

**Claims**

1. Process for the preparation of 1-methoxy-6-chlorohexyne-2 of formula:

$$CH_3O{-}CH_2{-}C{\equiv}C{-}CH_2{-}CH_2{-}CH_2{-}Cl$$

characterized in that the sodium salt of methyl-propargyl-ether of formula:

$$CH_3{-}O{-}CH_2{-}C{\equiv}C^{-}Na^{+}$$

is reacted in a solvent mixture consisting of tetrahydrofuran and an aprotic polar solvent in amounts ranging from 5 to 50% by volume based on tetrahydrofuran and at a temperature ranging from −10° to 10°C, with a substantially equimolar amount of 1-bromo-3-chloro-propane.

2. The process according to claim 1, characterized in that dimethylsulphoxide and/or hexamethyl-phosphorus triamide are used as aprotic polar solvents in admixture with tetrahydrofuran.

3. The process according to claim 1 or 2, characterized in that the aprotic polar solvent used in admixture with tetrahydrofuran is present in an amount of from 10 to 30% by volume based on tetrahydrofuran.

4. The process according to any of claims 1 to 3, characterized in that the sodium salt of methyl-propargylether is used in an amount of about 1 molar concentration with respect to tetrahydrofuran.

5. The process according to any of claims 1 to 4, characterized in that the sodium salt of methyl-propargyl-ether is prepared in situ by reacting the corresponding ether with sodium amide in anhydrous tetrahydrofuran.

6. The process according to any of claims 1 to 5, characterized in that the sodium salt of methyl-propargyl-ether is prepared in situ by reacting the corresponding ether with sodium amide in a predetermined amount of anhydrous tetrahydrofuran, whereupon 1-bromo-3-chloro-propane dissolved in the predetermined amount of aprotic polar solvent is added to this solution.

**Patentansprüche**

1. Verfahren zur Herstellung von 1 - Methoxy - 6 - chlor - hexin - 2 der Formel

$$CH_3O—CH_2—C\equiv C—CH_2—CH_2—CH_2—Cl$$

dadurch gekennzeichnet, daß das Natriumsalz von Methylpropargylether der Formel

$$CH_3—O—CH_2—C\equiv C^-Na^+$$

in einer Lösungsmittelmischung, die aus Tetrahydrofuran und einem aprotischen polaren Lösungsmittel in Mengen von 5 bis 50 Vol.-%, bezogen auf Tetrahydrofuran, besteht, bei einer Temperatur von −10 bis 10°C mit einer praktisch äquimolaren Menge 1-Brom-3-Chlorpropan umgesetzt wird.

2. Verfahren nach Anspruch 1, dadurch gekennzeichnet, daß Dimethylsulfoxid und/oder Hexamethyl-phosphor-triamid als aprotische polare Lösungsmittel in Mischung mit Tetrahydrofuran verwendet werden.

3. Verfahren nach Anspruch 1 oder 2, dadurch gekennzeichnet, daß das aprotische polare, in Mischung mit Tetrahydrofuran verwendete Lösungsmittel in einer Menge von 10 bis 30 Vol.-%, bezogen auf Tetrahydrofuran, anwesend ist.

4. Verfahren nach einem der Ansprüche 1 bis 3, dadurch gekennzeichnet, daß das Natriumsalz des Methylpropargylethers in einer Menge einer etwa 1-molaren Konzentration, bezogen auf Tetrahydrofuran, verwendet wird.

5. Verfahren nach einem der Ansprüche 1 bis 4, dadurch gekennzeichnet, daß das Natriumsalz des Methylpropargylethers in situ durch Umsetzung des entsprechenden Ethers mit Natrium-amid in Wasserfreiem Tetrahydrofuran hergestellt wird.

6. Verfahren nach einem der Ansprüche 1 bis 5, dadurch gekennzeichnet, daß das Natriumsalz des Methylpropargylethers in situ hergestellt wird, indem man den entsprechenden Ether mit Natriumamid in einer vorherbestimmten Menge an wasserfreiem Tetrahydrofuran umsetzt, worauf 1-Brom-3-chlor-propan, gelöst in der vorherbestimmten Menge an aprotischem polarem Lösungsmittel, zu dieser Lösung zugegeben wird.

**Revendications**

1. Procédé pour la préparation du 1-méthoxy-6-chloro-hexyne-2 de formule:

$$CH_3O—CH_2—C\equiv C—CH_2—CH_2—CH_2—Cl$$

caractérisé en ce que le sel de sodium du méthyl-propargyl-éther de formule:

$$CH_3—O—CH_2—C\equiv C^-Na^+$$

est amené à réagir dans un mélange de solvants formé de tétrahydrofuranne et d'un solvant polaire aprotique en des quantités comprises entre 5 et 50% en volume exprimées par rapport au tétrahydrofuranne et à une température comprise entre −10° et 10°C, avec une quantité pratiquement équimolaire de 1-bromo-3-chloro-propane.

2. Le procédé selon la revendication 1, caractérisé en ce que du diméthylsulfoxyde et/ou de l'hexaméthyl-phosphore triamide sont utilisés en tant que solvants polaires aprotiques en mélange avec le tétrahydrofuranne.

3. Le procédé selon la revendication 1 ou 2, caractérisé en ce que le solvant polaire aprotique utilisé, mélangé en association avec le tétrahydrofuranne, est présent en une quantité comprise entre 10 et 30% en volume exprimée par rapport au tétrahydrofuranne.

4. Le procédé selon une quelconque des revendications 1 à 3, caractérisé en ce que le sel de sodium du méthyl-propargyl-éther est utilisé en une quantité de l'ordre de la concentration molaire par rapport au tétrahydrofuranne.

5. Le procédé selon une quelconque des revendications 1 à 4, caractérisé en ce que le sel de sodium du méthyl-propargyl-éther est prépare in situ par réaction de l'éther correspondant avec l'amidure de sodium dans le tétrahydrofuranne anhydre.

6. Le procédé selon une quelconque des revendications 1 à 5, caractérisé en ce que le sel de sodium du méthyl-propargyl-éther est préparé in situ par réaction de l'éther correspondant avec l'amidure de sodium dans une quantité prédéterminée de tétrahydrofuranne anhydre, et ensuite le 1-bromo-3-chloro-propane dissous dans le quantité prédéterminée de solvant polaire aprotique est ajouté à cette solution.